(19) Europäisches Patentamt

European Patent Office

Office européen des brevets

(11) **EP 0 722 298 B1**

(12) **FASCICULE DE BREVET EUROPEEN**

(45) Date de publication et mention
de la délivrance du brevet:
**03.11.1999 Bulletin 1999/44**

(21) Numéro de dépôt: **94925525.1**

(22) Date de dépôt: **23.08.1994**

(51) Int. Cl.$^6$: **A61B 17/70**, A61B 17/02

(86) Numéro de dépôt international:
**PCT/FR94/01021**

(87) Numéro de publication internationale:
**WO 95/05786 (02.03.1995 Gazette 1995/10)**

(54) **MATERIEL ANCILLAIRE DE CORRECTION D'UNE DEFORMATION VERTEBRALE**

HILFSINSTRUMENTE FÜR DIE KORREKTUR EINER DEFORMIERTEN WIRBELSÄULE

ANCILLARY EQUIPMENT FOR CORRECTING A DEFORMED SPINE

(84) Etats contractants désignés:
**AT BE CH DE DK ES FR GB IT LI NL PT SE**

(30) Priorité: **27.08.1993 FR 9310291**
**07.02.1994 FR 9401440**
**15.07.1994 FR 9408794**

(43) Date de publication de la demande:
**24.07.1996 Bulletin 1996/30**

(73) Titulaires:
• **Fairant, Paulette**
**F-31170 Tournefeuille (FR)**

• **Martin, Jean-Raymond**
**F-31170 Tournefeuille (FR)**

(72) Inventeur: **MARTIN, Jean-Raymond**
**F-31170 Tournefeuille (FR)**

(74) Mandataire:
**Cabinet BARRE LAFORGUE & associés**
**95, rue des Amidonniers**
**31000 Toulouse (FR)**

(56) Documents cités:
**EP-A- 0 418 387      EP-A- 0 499 037**
**DE-C- 3 807 346      GB-A- 2 198 647**
**US-A- 4 112 935      US-A- 4 505 268**

## Description

L'invention concerne un matériel ancillaire pour la correction d'une déformation vertébrale préalablement à la mise en place d'une instrumentation rachidienne implantée telle qu'un dispositif d'ostéosynthèse, un dispositif intervertébral de traitement des instabilités ou une orthèse implantée dynamique de correction, ou autre.

On connaît déjà des dispositifs d'ostéosynthèse rachidienne permettant de traiter des déformations scoliotiques, constitues d'éléments d'ancrage sur les vertèbres tels que des crochets ou des vis intrapédiculaires et des tiges ou cadres fixés sur les éléments d'ancrage pour imposer une position relative aux différentes vertèbres. Ces dispositifs d'ostéosynthèse rigides, semi-rigides ou semi-souples réalisent une rigidification de la colonne vertébrale dans la position corrigée et sont le plus souvent associés à une arthrodèse intervertébrale avec ou sans greffe osseuse.

Ces dispositifs d'ostéosynthèse connus posent encore de nombreux problèmes en ce qui concerne la mise en place et la stabilité des éléments d'ancrage et lors de la fixation des tiges, plaques ou cadres, aux éléments d'ancrage qui doit être réalisée simultanément à la réduction de la déformation.

Ainsi, la réduction de la déformation lors de la pose du dispositif d'ostéosynthèse pose encore des problèmes. En effet, cette réduction de déformation doit pouvoir être effectuée au moment même de et par la pose de l'instrumentation vertébrale, et ce dans les trois dimensions. En particulier lors d'une scoliose, il convient non seulement de replacer les vertèbres dans un même plan sagittal, mais également de rétablir la cyphose et/ou la lordose tout en effectuant une dérotation des vertèbres. Les dispositifs d'ostéosynthèse de type Cotrel-Dubousset permettent de resoudre partiellement ce problème. Ils sont constitués de deux tiges bilatérales postérieures cintrées pendant l'intervention, immédiatement avant leur fixation dans des éléments d'ancrage, en fonction de la déviation latérale, puis tournées de 90° pour placer leur courbure dans un plan sagittal afin de retablir la cyphose ou la lordose et d'effectuer une dérotation des vertèbres au moins partiellement. La correction est limitée par le fait qu'elle est effectuée seulement par la tige de la concavité qui est placée et tournée en premier puis fixée aux éléments d'ancrage: elle stabilise ainsi la correction obtenue mais elle annule pratiquement tout potentiel correcteur de la tige de la convexité qui n'a qu'un effet stabilisateur par son insertion et sa fixation. Les deux tiges sont ensuite reliées l'une à l'autre par des tiges de traction transversale stabilisant l'ensemble en position obtenue. Même si la courbure des tiges peut être modifiée après leur insertion par des bras de levier, ces dernières peuvent assurer la cyphose ou la lordose voulue mais elles ne permettent pas de réaliser une dérotation vertébrale satisfaisante.

D'autres types de dispositifs d'ostéosynthèse sont employés pour traiter les déformations vertébrales et utilisent des fils sous-lamaires reliés à des tiges (Luque) ou à des cadres (Dove) ou relient des plaques ou des tiges en prenant appui sur les vertèbres par des crochets et/ou des vis intrapédiculaires. Outre les risques de complications neurologiques que présentent ces systèmes par les insertions de matériel intracanalaire, leur potentiel de correction des déformations vertébrales reste limité et incomplet.

Par ailleurs, on souhaite aussi disposer d'un matériel ancillaire adapté à la pose de nouvelles orthèses dynamiques qui preservent la mobilité physiologique des vetèbres et comportent des moyens de rappel élastique qui doivent être posés tendus et dont les caracteristiques doivent être déterminées préalablement à cette pose pour assurer le maintien de la correction obtenue et permettre des mouvements à la zone vertébrale instrumentée.

Les matériels ancillaires connus sont constitués par de simples pinces à deux branches articulées et exerçant des forces de détraction ou de compression, ne permettant pas de résoudre les différents problèmes de correction totale des déformations vertébrales.

Kluger a décrit un matériel ancillaire pour la correction d'un écrasement vertébral (traumatique ou tumoral) en prenant appui sur les deux vertèbres adjacentes dont il effectue l'espacement par un dispositif détracteur-compresseur: la hauteur de la vertèbre considérée est ainsi reconstituée. Ce matériel ne peut permettre la correction d'une déviation vertébrale étant donné qu'il ne prend appui que sur deux vertèbres dont il ne peut réaliser que le rapprochement ou l'éloignement. Il maintient la position obtenue jusqu'à l'insertion d'un matériel de liaison qui va assurer la fixation définitive des vertèbres dans cette position.

Par ailleurs Bookwalter a décrit un cadre vertébral qui sert de support à des écarteurs des muscles paravertébraux. Il n'a pour but et pour effet que d'écarter les muscles paravertébraux pour améliorer l'exposition des vertèbres durant la chirurgie : en aucun cas ce matériel n'a d'action sur les vertèbres ellesmêmes.

On connaît aussi des matériels externes volumineux de réduction des deformations vertébrales constitués de harnais et/ou halos et/ou ceintures associés à des dispositifs détracteurs (moteurs, poids...). Ces matériels sont peu précis, ne sont pas souples d'emploi, et ne permettent pas une correction importante et précise des positions des vertèbres dans les trois dimensions.

Ainsi, aucun matériel ancillaire connu ne permet la maîtrise avec précision des positions des vertèbres dans les trois dimensions de l'espace dans les plans frontal, sagittal et horizontal.

US-4 505 268 décrit un cadre comprenant des dispositifs de correction fixés sur des rails longitudinaux. Chaque dispositif de correction comprend un arbre fileté fixé sur l'un des rails longitudinaux, et un arbre de correction portant un crochet. Cet arbre de correction a une longueur fixé. Ce dispositif ne permet pas des mouvements d'ajustement et un maintien de chaque d'extrémité d'action selon les deux directions orthogonales horizontales frontale et sagittale. En effet, chaque extrémité d'action ne peut être ajustée que selon l'une de ces deux directions.

L'invention vise donc à pallier ces inconvénients et à proposer un matériel ancillaire permettant de corriger et/ou de maintenir avec une grande précision et dans les trois dimensions la forme et/ou les efforts exercés entre les vertèbres avant et pendant la pose d'une instrumentation rachidienne implantée.

L'invention vise également à proposer un matériel ancillaire permettant la pose d'une orthèse vertébrale implantée dynamique. En particulier, l'invention vise aussi à proposer un matériel ancillaire qui permet de mesurer les déplacements et les forces nécessaires au maintien de la correction et qui seront donc imposés à l'instrumentation posée d'ostéosynthèse ou d'orthèse dynamique.

L'invention vise également à proposer un matériel ancillaire simple dans sa structure et son utilisation et relativement peu encombrant vis-à-vis du champ opératoire.

Pour ce faire, L'invention concerne un matériel ancillaire permettant d'exercer et de maintenir des contraintes sur une portion de la colonne vertébrale en vue de corriger et/ou de maintenir la forme et/ou les efforts exercés sur les vertèbres avant et pendant la pose d'une instrumentation rachidienne implantée., comprenant au moins deux extrémités d'action destinées à coopérer respectivement avec au moins deux vertèbres distinctes, en s'appuyant, par leur branche correctrice correspondante, et par leur base amovible, sur des rails longitudinaux et parallèles d'un support en forme de cadre, et également des moyens pour modifier et maintenir les positions relatives des extrémités d'action, caractérisé en ce que lesdits moyens sont adaptés pour modifier et maintenir les positions relatives des extrémités d'action selon chacune, isolément ou en association, des trois directions orthogonales. L'invention concerne aussi un matériel ancillaire permettant d'exercer et de maintenir des contraintes sur une portion de la colonne vertébrale en vue de corriger et/ou de maintenir la forme et/ou les efforts exercés sur les vertèbres avant et pendant la pose d'une instrumentation rachidienne implantée, comprenant un cadre rectangulaire destiné à entourer la region vertébrale à instrumenter et formé de deux petits cotés transversaux, supérieur et inférieur, et de quatre barres parallèles longitudinales, deux de chaque côté de la colonne vertébrale sur lesquelles vont coulisser des branches de correction. Selon l'invention, ces branches de correction comportent une base qui permet de fixer transversalement chaque branche sur les deux barres longitudinales du cadre tout en conservant la totale liberté de déplacement et de glissement longitudinal. Sur la base s'appuie la partie correctrice de chaque branche dont l'extrémité est destinée à coopérer avec le côté correspondant de la vertèbre choisie: cette extrémité va assurer le déplacement dans le plan horizontal du côté vertébral instrumenté et selon une direction frontale et/ou sagittale sous l'action respective de deux poignées actionnant ses pas de vis situés sur des axes s'appuyant sur la base. Les déplacements ainsi realisés sont evalués par des échelles métriques alors que les forces sont mesurées par des dynamomètres correspondants.

Selon l'invention, les forces de détraction et/ou de compression rentrant en jeu dans la correction des déformations vertébrales sont évaluées par des éléments de mesure que sont soit une pince dynamométrique à deux branches soit un distracteur dynamométrique du type pied à coulisse, chacun des deux effectuant son action sur la base des branches de correction. Chaque branche de correction est indépendante d'un même côté du cadre et se trouve indirectement reliée, par l'intermédiaire de la vertèbre sur laquelle elle s'appuie, à la branche correctrice située, sur la même vertèbre, du côté opposé de la colonne vertébrale. Donc chaque vertèbre choisie pour être instrumentée subit sur chacun de ses côtes droit et gauche les actions différentes de deux branches de correction situées respectivement sur le cadre du coté droit et gauche.

L'invention concerne aussi un matériel ancillaire comportant en combinaison tout ou partie des caractéristiques mentionnées ci-dessus ou ci-après.

D'autres caractéristiques et avantages de l'invention apparaitront à la lecture de la description détaillée suivante qui se réfère aux dessins annexés dans lesquels:

- la figure 1 est une vue schématique postérieure d'un cadre ancillaire avec plusieurs branches de correction vertébrale,
- la figure 2 est une vue schématique en coupe latérale selon l'axe III-III d'une branche dynamométrique de correction du cadre,
- la figure 3 est une vue schématique supérieure d'une branche dynamométrique de correction du cadre,
- la figure 4 est une vue schématique latérale d'une pince dynamométrique, élément de mesure des forces en détraction et/ou en compression,
- la figure 5 est une vue schématique latérale d'un élément dynamométrique de mesure des forces en détraction et/ou en compression,
- les figures 6 et 7 sont des vues des phases de correction d'une déformation vertébrale de type scoliotique et de l'insertion d'une orthèse vertébrale interne dynamique dans le but de maintenir cette correction tout en permettant une certaine mobilité vertébrale.

Dans tout le texte, et sauf indication contraire, le terme "vertical" désigne la direction axiale de la colonne vertébrale qui ne correspond pas toujours à la direction verticale absolue puisque la colonne présente des courbures en cyphose et/ou en lordose. De même, le terme "horizontal" désigne toute direction contenue dans le plan perpendiculaire à la direction verticale, le terme "sagittal" désigne tout plan contenant les directions verticale et horizontale antéro-postérieure, et le terme "frontal" désigne tout plan contenant les directions verticale et horizontale latérales. Ces termes sont donc utilisés en référence à chaque vertèbre et non, dans l'absolu, en référence au patient.

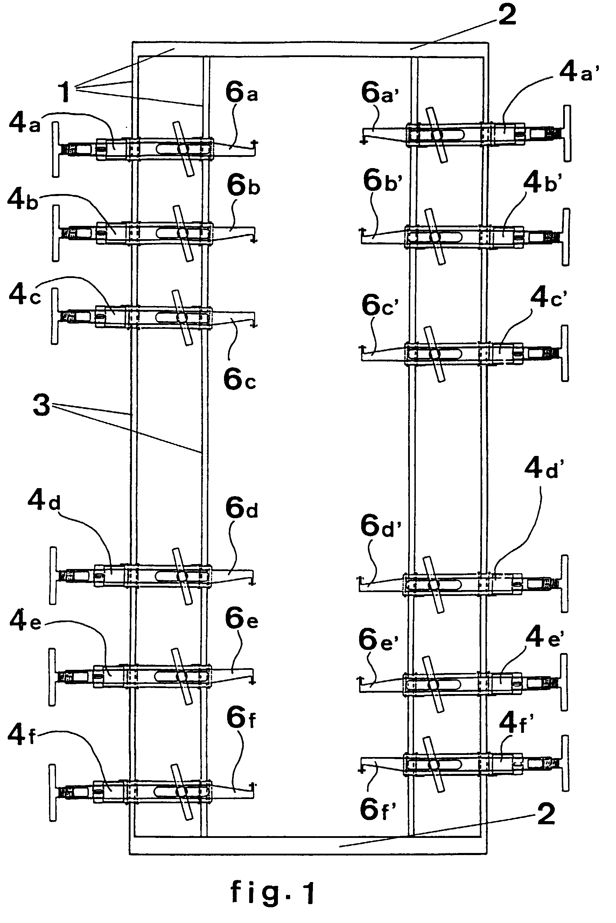

La figure 1 représente un cadre ancillaire selon l'invention permettant notamment la correction d'une déformation vertébrale du type scoliotique et/ou hyper-hypocyphotique et/ou hyper-hypolordotique. Ce cadre rectangulaire(1) présente:

- 2 petits côtés (2) transversés positionnés à la partie supérieure et inférieure de la colonne vertébrale. Ces petits côtés sont à priori fixés mais peuvent éventuellement être télescopiques afin d'ajuster leur longueur à l'écartement nécessaire entre les grands côtés.

- 2 grands côtés(3) longitudinaux constitués par 2 barres parallèles, de chaque côté de la colonne vertébrale, sur lesquelles vont coulisser les bases(5) des branches de correction(4). L'écartement des deux grands côtés(3) est fixé, symétrique et correspond à celui des cylindres(8) de fixation des bases(5) des branches de correction(4) : il est de l'ordre de 10 à 15cm. La longueur des côtés longitudinaux(3) doit être supérieure à celle de la colonne instrumentée et justifié la disponibilité de 2 ou plusieurs longueurs de cadre afin de pouvoir utiliser celle adaptée à la taille du sujet et à la hauteur (nombre de niveaux) de la colonne à corriger.

Selon l'invention, ce cadre se fixe par ses côtés et/ou par ses extrémités (de préférence dans les angles) à la table opératoire ou au support sur lequel repose le patient en décubitus ventral. Cette fixation se fait par l'intermédiaire de bras articulés (de deux à quatre en moyenne) qui sont bloqués pour immobiliser le cadre une fois que ce dernier a été positionné par rapport au patient. Les bras articulés se fixent sur le cadre au moyen de pinces autobloquantes.

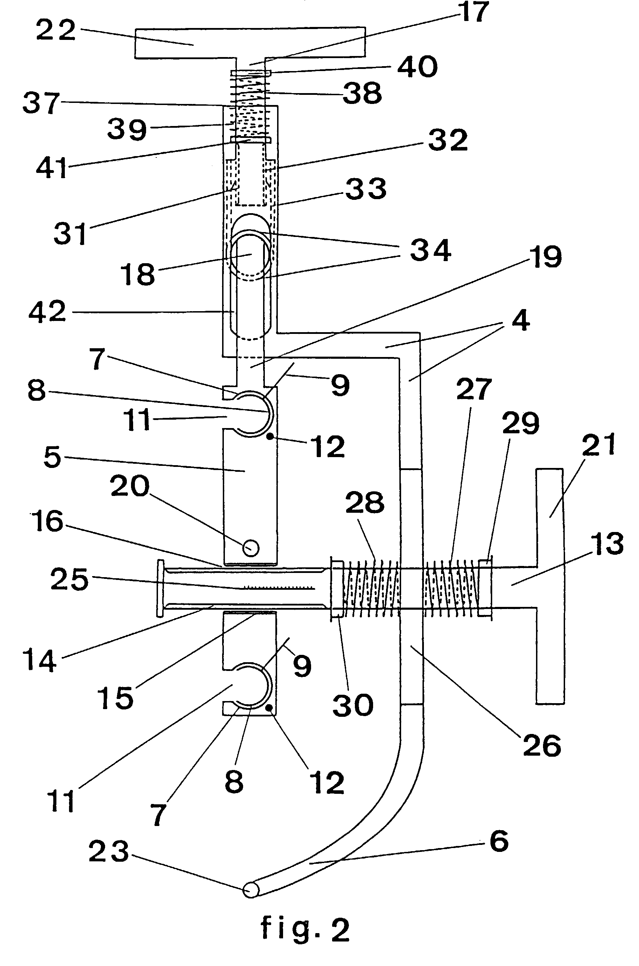

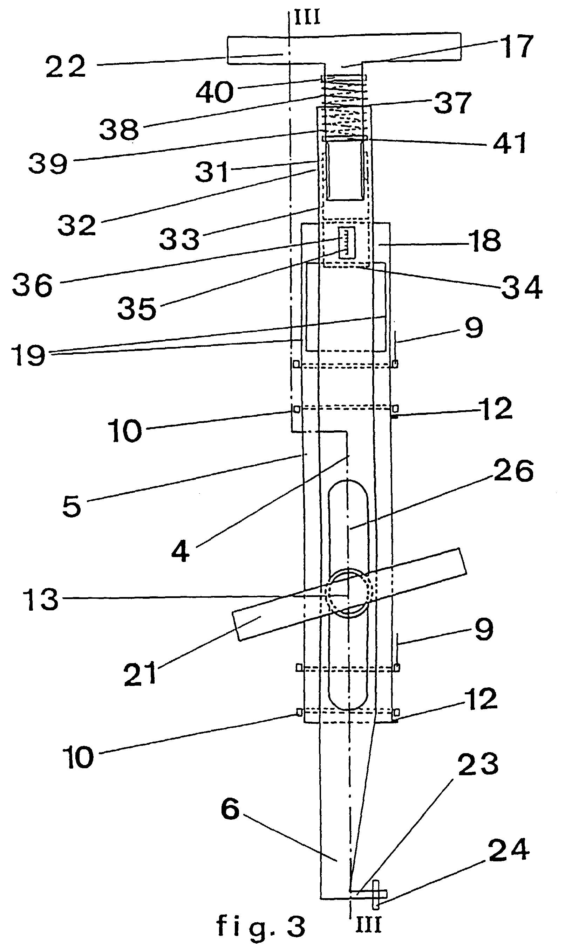

Les figures 2 et 3 représentent les branches de correction, selon l'invention, qui sont constituées de 2 parties: une première partie inférieure(5) ou base qui sert d'une part à la fixation de la branche sur le cadre tout en lui permettant des mouvements libres de déplacement longitudinaux et d'autre part d'appui à la deuxième partie dite "correctrice" (4). Cette partie correctrice (4) se fixe à son extrémité libre(6) sur l'élément d'ancrage, telle une plaque-crochets, (et donc sur la vertèbre) pour agir par l'intermédiaire de 2 poignées distinctes(21, 22).

Avantageusement selon l'invention, les branches de correction ne font pas partie intégrante du cadre : elles sont amovibles et peuvent donc être ajoutées ou enlevées selon les besoins. Une branche de correction correspond à un côté d'une vertèbre instrumentée.

Selon l'invention, les branches (4) se fixent par leur base(5) sur les deux tiges parallèles (3) du cadre de chaque côté de la colonne par deux logements cylindriques (7) prévus à leur partie inférieure. Chaque logement cylindrique (7) renferme un autre cylindre concentrique (8), libre en rotation axiale mais non en translation longitudinale, qui va tourner à l'intérieur au premier (7) par l'action exercée sur une manette extérieure(9). La translation longitudinale du cylindre interne(8) est limitée par un renflement périphérique (10) de ce cylindre à chacune de ses extrémités et à l'extérieur du cylindre principal (7) de la base. Sur ce renflement externe(10) du cylindre (8) prend appui la manette transversale(9) qui va en permettre la rotation. Ces deux cylindres(7 et 8) sont ouverts(11) longitudinalement à leur partie inférieure pour permettre la pénétration de chaque tige longitudinale(3) correspondante du cadre. La largeur de cette ouverture(11) est juste supérieure au diamètre de la tige longitudinale(3) du cadre qui vient s'y insérer. De même le diamètre interne du cylindre(8) est juste supérieur à celui de la barre(3) qu'il va retenir sans la fixer. Le blocage s'effectue alors par rotation du cylindre central(8) qui ferme ainsi la fenêtre inférieure(11). La base(5) de la branche de correction(4) ne peut donc plus quitter les deux tiges parallèles(3) du cadre mais peut y avoir des mouvements libres de glissements longitudinaux selon l'axe vertical. Un ergot(12) placé sur le côté de la base(5) permet de bloquer la manette(9) en position "fermée" du cylindre central(8).

Selon l'invention, la tige sagittale et horizontale(13) de la branche correctrice(6) se déplace perpendiculairement à sa base(5) qu'elle traverse par rotation. Son déplacement s'effectue à l'aide d'un filetage(14) qui correspond à un taraudage(15) realisé dans l'orifice(16) sagittal et horizontal correspondant de la base(5).

Selon l'invention, la tige frontale et horizontale(17) de la branche correctrice(4) se déplace en prenant appui sur un axe vertical et frontal(18), de la base(5), situé dans son prolongement horizontal. Cet axe(18) est soutenu et fixé à chacune de ses extrémités par deux prolongements(19) parallèles de la base(5) qui permettent dans leur écartement le déplacement frontal et horizontal de la branche correctrice(4).

Selon l'invention, les appuis des éléments de mesure en détraction-compression se font sur les faces latérales situées dans le plan horizontal de la base(5) au niveau d'orifices aveugles(20) permettant de recevoir les ergots(44, 49) des éléments de mesure en détraction-compression. Ces orifices sont situés à mi-distance des 2 cylindres(8) de fixation sur les 2 tiges longitudinales(3) parallèles du cadre: l'action des éléments de mesure en détraction-compression se fait de manière équilibrée sur la base qui se déplace verticalement dans le plan frontal sur les 2 tiges(3) longitudinales du cadre.

Selon l'invention, la branche correctrice proprement dite(4) se déplace horizontalement dans le plan sagittal et dans le plan frontal sous l'action de 2 axes(13, 17) appuyés sur la base(5), et mis en mouvement par 2 poignées(21, 22). Elle se fixe, par exemple par un ergot(23) à son extrémité dynamique(6), sur la face supérieure ou inférieure du moyen d'ancrage de la vertèbre instrumentée. Cet ergot(23) comporte à son extrémité un écrou(24) qui va réaliser la stabilité de la liaison "élément d'ancrage-branche correctrice". Cet ergot(23) peut être utilisé lorsqu'une plaque-crochets est employée comme moyen d'ancrage sur la vertèbre instrumentée; tout autre moyen de fixation (crochet, pince, fourche, etc.) peut être utilisé à cette extrémité dynamique(6) de la branche correctrice(4) pour prendre appui sur la vertèbre en fonction de l'ancrage du système vertébral implanté.

Selon l'invention, le déplacement horizontal et sagittal de l'ergot(23) de l'extrémité dynamique(6) de la branche correctrice(4) est réalisé par une tige sagittale(13) pourvue, à son extrémité, d'un filetage(14) engagé dans un taraudage(15) de la base(5). La position de la branche correctrice(4) par rapport à la base(5) est repérée par une échelle graduée(25) sur la tige(13) ou par un moyen électronique non représenté. Cette branche correctrice(4) comporte une lumière oblongue(26) traversée par la tige de commande horizontale et sagittale(13). Cette lumière oblongue(26) s'étend selon une direction orthogonale à la direction verticale passant par les rails longitudinaux(3) du cadre et à l'axe de la tige sagittale(13). Ainsi un déplacement de cette branche correctrice(4) par rapport à l'axe horizontal et sagittal est possible. La lumière oblongue(26) de cette tige correctrice(4) est emprisonnée entre 2 ressorts(27, 28) dont les extrémités opposées s'appuient sur des capteurs dynamométriques(29, 30) portes par la tige horizontale et sagittale(13). Ces capteurs dynamométriques(29, 30) fournissent une mesure des efforts impartis au téton(23) de l'extrémité dynamique(6) de la branche correctrice(4) dans la direction sagittale horizontale. En tournant la poignée(21) de cette tige sagittale(13) on modifie la position de la branche correctrice(4) dans la direction sagittale-horizontale par rapport au plan frontal contenant les rails longitudinaux(3) du cadre(1).

Selon l'invention, le déplacement horizontal et frontal de l'ergot(23) de l'extrémité libre(6) de la branche correctrice(4) est assuré par une tige de commande frontale(17) qui permet de réaliser les mouvements de cette branche correctrice(4) dans la direction frontale-horizontale perpendiculaire à la direction verticale passant par les rails longitudinaux(3) du cadre(1). Cette tige de commande frontale(17) comporte une extrémité filetée(31) engagée dans un taraudage(32) ménagé dans un palier(33) comprenant un cylindre(34) entourant l'axe vertical(18) situé dans le prolongement de la base(5) et solidarisé à celle-ci par ses 2 prolongements parallèles(19). Le cylindre(34) comporte une échelle graduée(35) visible à travers une lumière(36) de la branche correctrice(4) et qui permet d'évaluer le déplacement de cette branche(4). L'extrémité(37) de cette branche(4) opposée au téton(23) coulisse autour de la tige de commande frontale(17) et est emprisonnée autour de 2 ressorts(38, 39) dont les extrémités opposées s'appuient sur des capteurs dynamométriques(40, 41) portés par la tige frontale(17). Ces ressorts et ces capteurs forment donc une butée axiale dans les 2 sens pour l'extrémité(37) de cette branche correctrice(4) par rapport à la tige de commande frontale(17). Le déplacement de la branche correctrice(4) dans la direction frontale horizontale par rapport à la tige de commande sagittale(13) est autorisé grâce à une lumière oblongue(26). L'axe vertical(18) de la base(5) traverse la branche correctrice(4) à travers une lumière oblongue(42) ménagée axialement dans cette branche pour permettre les mouvements relatifs dans la direction frontale horizontale. En tournant la poignée(22) de la tige frontale(17) on modifie donc la position du téton(23) de l'extrémité dynamique(6) de la branche(4) dans le plan frontal.

La force de correction appliquée à la vertèbre correspond à une traction dans la concavité et à une poussée dans la convexité: il en résulte dans chaque cas une compression d'1 des 2 ressorts longitudinaux(38 ou 39) situés sur la tige de commande frontale, la force de compression étant évaluée par le capteur dynamométrique(40 ou 41) correspondant qui transmet directement ses informations à l'ordinateur.

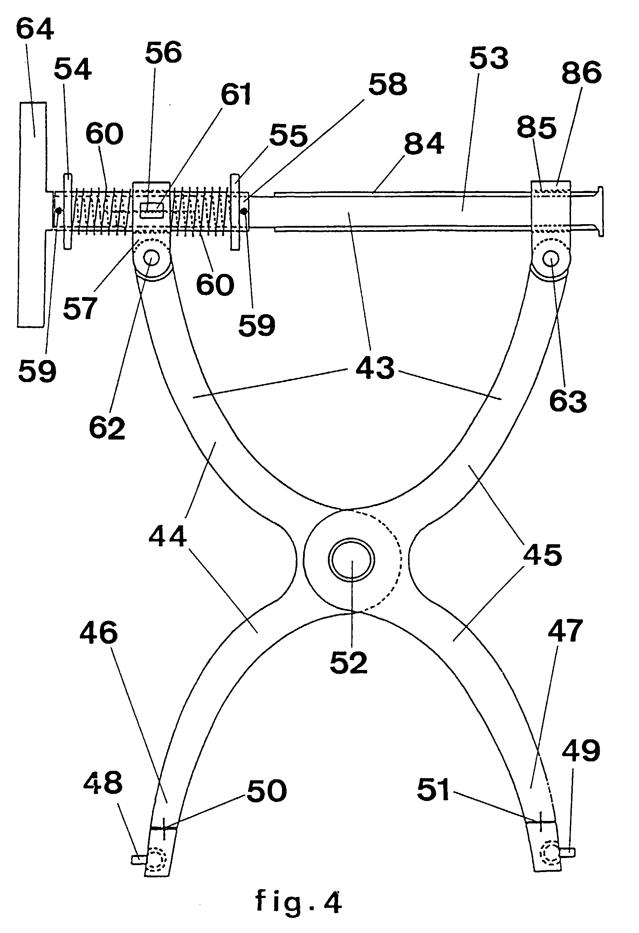

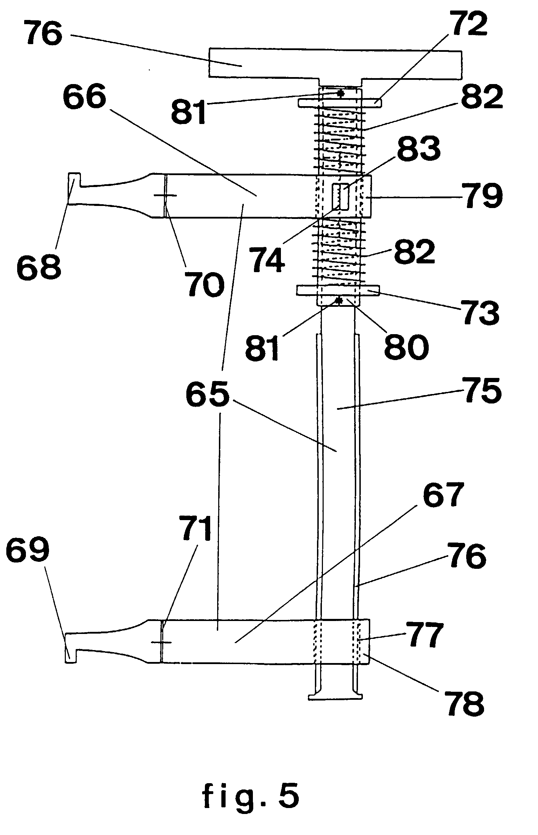

Les figures 4 et 5 représentent, selon l'invention, les éléments de mesure(43, 65) en détraction-compression qui ne vont réaliser aucun déplacement puisque la correction complète dans les trois plans est obtenue par les branches(4) du cadre(1). Ces éléments permettent la mesure des forces en détraction ou en compression qui vont être demandées aux ressorts d'une orthèse dynamique; ces ressorts sont interposés entre les vertèbres et s'appuient sur les moyens d'ancrage. Les forces produites par ces ressorts vont donc réduire d'autant celles demandées aux tiges de liaison: ainsi la réduction du diamètre des tiges de liaison accentue la souplesse résiduelle du système. Ces forces en détraction-compression sont des "forces de repos" puisqu'elles ne réalisent aucun déplacement des branches sur lesquelles elles sont appliquées.

Cette mesure se fait donc dans le plan frontal et selon la direction verticale des deux tiges parallèles longitudinales(3) du cadre(1).

Ces éléments(43, 65) permettent en outre la mesure de la distance entre deux vertèbres instrumentées non adjacentes, mesure qui correspond à la corde de l'arc de la courbure en cypho-lordose choisie pour la zone vertébrale considérée.

La figure 4 représente, selon l'invention, une pince ancillaire(43) qui comporte deux extrémités d'action(46 et 47) destinées à coopérer avec les bases(5) des branches correctrices(4) sur le cadre(1). Chacune des extrémités d'action(46 et 47) de la pince(43) est formée d'un téton(48, 49) destiné à être engagé dans l'orifice(20) de la base(5) de la branche correctrice(4), afin d'agir en compression ou en détraction sur cette base(5), à mi-distance entre les deux barres parallèles(3) du cadre(1): pour ce faire chaque téton(48, 49) peut être orienté vers le haut ou vers le bas par rotation autour de leurs axes(50, 51).

Selon l'invention la pince comporte également des moyens dynamométriques(54 et 55) de mesure des forces imparties sur les tetons d'extrémité(48, 49) pour maintenir leurs positions relatives. Egalement, la pince comporte un moyen de mesure(56), métrique ou électronique, des déplacements des tétons d'extrémité(48, 49) lors des modifications de leur position relative.

Chaque pince est composée de deux branches(44, 45), portant les tétons(48, 49) à leur extrémité libre, qui sont articulées l'une à l'autre autour d'un axe horizontal(52), orthogonal à la direction passant par les deux tétons(48, 49). Les deux branches(44, 45) sont commandées dans leurs mouvements relatifs par une tige de commande(53) munie d'une poignée(64) de manoeuvre.

Une tige de commande(53) comporte un filetage(84) coopérant avec un taraudage(85) d'une extrémité(86) en forme de manchon de la branche (45) opposée au téton(49). L'extrémité(57) de la branche(44) opposée au téton(48) est en forme de manchon coulissant autour d'un cylindre(58) solidaire de la tige de commande verticale(53) mais dont la position en translation par rapport à cette tige(53) peut être ajustée grâce à des vis de blocage(59), permettant de régler en position de repos l'écartement des tétons(48, 49). Ce manchon(57) est emprisonné entre deux ressorts de compression(60) prenant appui à leurs extrémités opposées sur des capteurs dynamométriques(54, 55) portés par le cylindre(58) et donc par la tige de commande verticale(53). Les ressorts(60) et leurs capteurs(54, 55) forment ainsi une butée axiale dans les deux sens pour l'extrémité(57) de la branche(44) par rapport à la tige de commande(53). Le manchon(57) comporte également une lumière(61) permettant la lecture d'une échelle graduée(56) gravée sur la tige(53). Les manchons (86, 57) d'extrémité des branches(45, 44) coopérant avec la tige de commande verticale(53) sont articulés à ces branches(45, 44) autour d'un axe(63, 62) parallèle à l'axe(52) d'articulation des branches(44, 45) entre elles. Lorsque la poignée(64) est tournée, les tétons d'extrémité(48, 49) sont écartés ou rapprochés l'un de l'autre. Si les tétons(48, 49) ne supportent pas de forces dans la direction verticale, le manchon(57) reste à mi-distance des deux capteurs(54, 55), les ressorts(60) n'étant pas activés. Si au contraire une force est nécessaire pour déplacer les tétons(48, 49), l'un des ressorts(60) est activé en compression pour équilibrer cette force et permettre la modification de position. Un des capteurs(54, 55) délivre alors un signal électrique proportionnel à cette force.

La figure 5 represente, selon l'invention, un pied à coulisse ancillaire(65) de mesure de forces en détraction-compression qui comporte deux extrémités d'action(66 et 67) destinées à coopérer avec les bases(5) des branches correctrices(4) sur le cadre(1). Chacune des extrémités d'action(66 et 67) du pied à coulisse(65) est formée d'un téton(68, 69) destiné à être engagé dans l'orifice(20) de la base(5) de la branche correctrice(4), afin d'agir en compression ou en détraction sur cette base(5), à mi-distance entre les deux barres parallèles(3) du cadre(1): pour ce faire chaque téton(68, 69) peut être orienté vers le haut ou vers le bas par rotation autour de leurs axes(70, 71).

Selon l'invention, le pied à coulisse(65) comporte des moyens dynamométriques(72, 73) de mesure des forces imparties sur les tétons d'extrémité(68, 69) pour maintenir leurs positions relatives. Egalement, le pied à coulisse(65) comporte un moyen de mesure(74) des déplacements des tétons d'extrémité(68, 69) lors des modifications de leur position relative.

Chaque pied à coulisse(65) est composé de deux branches(66, 67), portant les tétons(68, 69) à leur extrémité libre et qui restent parallèles dans leurs déplacements qui sont commandés par une tige orthogonale de commande(75) munie d'une poignée(76) de manoeuvre.

Une tige de commande(75) comporte un filetage(76) coopérant avec un taraudage(77) d'une extrémité(78) en forme de manchon de la branche(67) opposée au téton(69). L'extrémité(79) de la branche(66) opposée au téton(68) est en forme de manchon coulissant autour d'un cylindre(80) solidaire de la tige de commande verticale(75) mais dont la position en translation par rapport à cette tige(75) peut être ajustée grâce à des vis de blocage(81), permettant de régler en position de repos l'écartement des tétons(68, 69). Ce manchon(79) est emprisonné entre deux ressorts de compression(82) prenant appui à leurs extrémités opposées sur des capteurs dynamométriques(72, 73) portés par le cylindre(80) et donc par la tige de commande verticale(75). Les ressorts(82) et leurs capteurs(72, 73) forment ainsi une butée axiale dans les deux sens pour l'extrémité(79) de la branche(66) par rapport à la tige de commande(75). Les déplacements du manchon(79) sont évalués soit par la lecture, à travers une lumière(83), d'une échelle métrique graduée(74) gravée sur la tige(75), soit par un moyen électronique non représenté. Lorsque la poignée(76) est tournée, les tétons d'extrémité(68, 69) sont écartés ou rapprochés l'un de l'autre. Si les tétons (68, 69) ne supportent pas de force dans la direction verticale, le manchon(79) reste à mi-distance des deux capteurs(72, 73), les ressorts(82) n'étant pas activés. Si au contraire, une force est nécessaire pour déplacer les tétons(68, 69), l'un des ressorts(82) est activé en compression pour équilibrer cette force et permettre la modification de position. Un des capteurs(72, 73) délivre alors un signal électrique proportionnel à cette force.

Ces deux éléments(43 et 65) de mesure en détraction-compression permettent de compléter l'action des branches correctrices(4) fixées sur le cadre(1): ils réduisent les forces transversales horizontales, sagittales ou frontales, de ces branches et permettent de mesurer les forces demandées en détraction ou en compression aux ressorts d'une orthèse vertébrale interne dynamique qui seront placés entre les éléments d'ancrage.

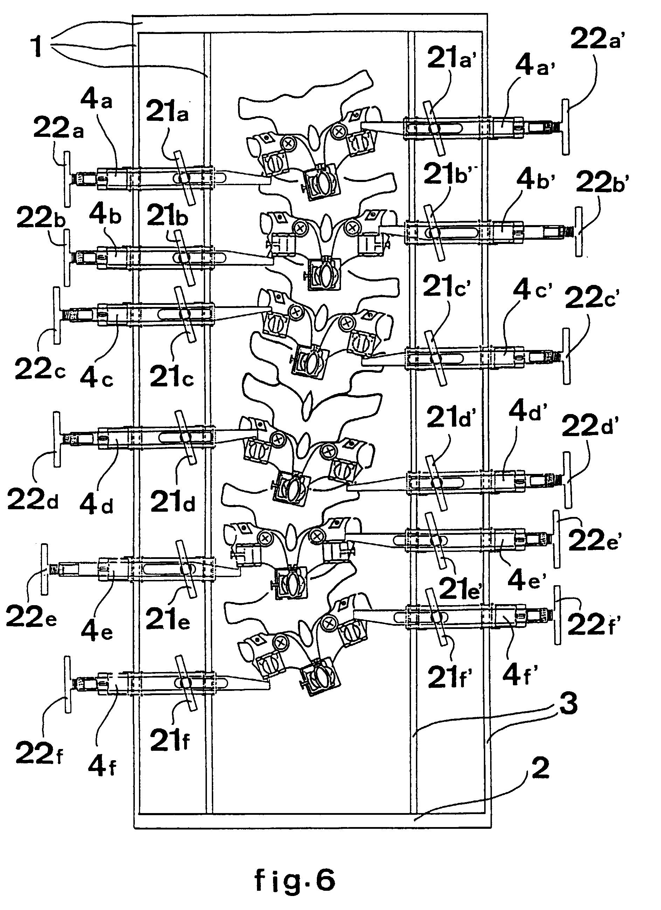

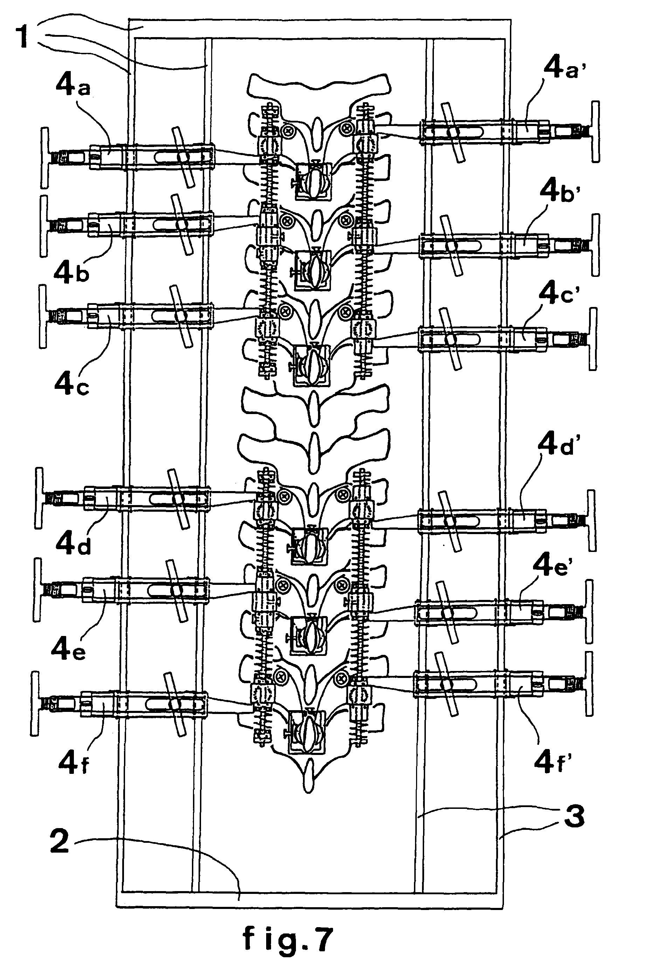

Les figures 6 et 7 représentent, un procédé d'utilisation du matériel ancillaire, selon l'invention, pour la correction d'une déformation vertébrale du type scoliotique et le maintien de cette correction par l'insertion d'une orthèse vertébrale interne dynamique.

Le patient est positionné en décubitus ventral sur appuis thoraciques et pelviens classiques et l'abord vertébral postérieur et médian est fait sur la longueur de la zone à instrumenter. Après dégagement des masses musculaires paravertébrales la fixation du matériel d'ancrage est effectuée sur les vertèbres choisies afin de réaliser soit une ostéosynthèse soit la pose d'une orthèse vertébrale dynamique.

Ce matériel d'ancrage doit comporter une zone d'attache pour l'extrémité correctrice des branches du cadre qui vient s'y fixer. Le matériel d'ancrage est habituellement bilatéral mais si la correction s'adresse à une déviation minime il peut être placé unilatéralement.

Le cadre(1) est ensuite positionné avec ses deux barres(3) longitudinales parallèles et bilatérales de part et d'autre de la zone opératoire. La longueur du cadre est choisie de manière à ce que les deux petits côtés transversés(2) supérieur et inférieur soient placés au-delà des extrémités supérieure et inférieure de l'incision.

Les bras articules stériles, solidarisés soit à la table opératoire soit au support du patient, permettent de fixer fermement le cadre(1) dans l'espace: les rails longitudinaux ne doivent pas être en contact avec la paroi postérieure du patient, que ce soit au niveau thoracique ou au niveau fessier, afin de permettre la libre mobilité des bases(5) des branches correctrices(4) sur ces rails(3).

Chaque base(5a, 5b, 5c....à gauche ou 5a', 5b', 5c',...à droite) de la branche correctrice(4a, 4b, 4c,... à gauche ou 4a', 4b', 4c',... à droite) est posée sur les deux rails longitudinaux(3) du cadre(1) en regard de chaque vertèbre et ensuite verouillée par la rotation des cylindres(8) correspondants de la base(5). Deux branches correctrices(4), une de chaque côté, sont positionnées sur une même vertèbre instrumentée par un moyen d'ancrage bilatéral; eventuellement d'un seul côté (habituellement celui de la concavité) en cas de faible déviation instrumentée unilatéralement.

Par la rotation des poignées(21, 22) respectivement des tiges sagittales-horizontales(13) et frontales-horizontales(17), les extrémités dynamiques(6) de chaque branche correctrice(4) sont positionnées au contact des moyens d'ancrage où elles sont fixées par un écrou(24) sur le téton(23) comme dans le cas d'une orthèse dynamique. La figure 6 montre le système en équilibre et en position de repos avant toute correction.

La correction se fait de manière progressive par la rotation des tiges sagittales-horizontales(13) et frontales-horizontales(17):

La rotation par leurs poignées(22) des tiges frontales-horizontales(17) doit se faire simultanément de chaque côté de la vertèbre instrumentée: en effet l'écartement entre les deux moyens de fixation des extrémités dynamiques(6) des branches correctrices(4) est constant sur la vertèbre. Le déplacement de chaque branche correctrice(4) est donc symétrique: il en résulte un équilibre entre les forces de traction de la concavité et les forces de pression de la convexité de la courbure. Si dans la convexité, la force de pression enregistrée par le dynamomètre(40) est faible, par rapport à celle de la concavité enregistrée par le dynamomètre(41), ou si a fortiori nous enregistrons une force de traction dans la convexité, cela signifie que la poussée de la branche correctrice(4) dans la convexité n'est pas suffisante par rapport à la traction exercée par la branche correctrice(4) de la concavité.

Le système est considéré en équilibre, que la correction totale soit ou non obtenue en rapport avec des problèmes mécaniques vertébraux ou des problèmes de souffrance neurologique, lorsque les forces de traction de la concavité et de pression de la convexité sont identiques ou de valeurs très proches. La rotation, par leurs poignées(21), des tiges sagittales-horizontales(13) ne se fait pas de façon symétrique comme celle des tiges frontales-horizontales(17). Chaque tige frontale-horizontale(17) réalise un double déplacement de chaque côté de la vertèbre instrumentée dans deux plans:

- sagittal, en cyphose ou lordose
- horizontal, pour la correction de la courbure dans le plan frontal.
  Comme la position de la vertèbre à corriger n'est pas symétrique par rapport à son centre dans ces deux plans, chaque côté de cette vertèbre instrumentée ne va pas subir le même déplacement lors de la correction: dans la concavité, le déplacement est plus important, jusqu'à la position de correction voulue, que du côté de la convexité. En fait c'est la correction de la cypho-lordose de manière symétrique entre la concavité et la convexité qui va permettre, associée à la correction de la courbure dans le plan frontal, de réaliser la dérotation totale de la vertèbre.

Le déplacement dans le plan sagittal et horizontal de chaque côté vertébral engendre donc une correction totale de la rotation vertébrale par une action bilatérale qui crée un volant de dérotation. La position de cyphose ou de lordose voulue est obtenue de manière précise par calcul de la flèche de la courbure désirée et reproduite entre la branche correctrice centrale (et donc du sommet de la courbure) et les deux branches correctrices positionnées sur les vertèbres adjacentes. L'angle α de Cobb de cypho-lordose voulue et déterminé pré-opératoirement permet le calcul de la flèche de la courbure en connaissant la corde de l'arc (distance entre les vertèbres extrèmes donnée par les éléments de mesure(43, 65) et selon la formule:

$$\text{Flèche} = \text{Tan}\,\frac{\alpha}{4} \times \frac{\text{corde}}{2}.$$

La correction totale étant obtenue, nous avons les valeurs des forces nécessaires à cette correction qui nous sont données sur chaque pince par deux des dynamomètres(29, 30, 40, 41). La réduction des forces demandées aux tiges de liaison va permettre d'en réduire le diamètre et donc la rigidité, leur conservant ainsi le maximum de souplesse pour faciliter la liberté de mouvement de la colonne vertébrale. Cet effet va être obtenu par l'insertion de ressorts entre les vertèbres afin de réaliser soit une force en détraction dans la concavité soit une force en compression dans la convexité. Ces ressorts ne doivent pas effectuer de déplacement des vertèbres sur lesquelles ils appliquent leurs efforts puisque la correction totale de la déviation a déjà été obtenue.
Les éléments de mesure(43, 65) en détraction ou en compression sont mis en place respectivement dans la concavité et dans la convexité et s'appuient sur les bases(5) des branches correctrices(4) de deux vertèbres adjacentes ou non: la rotation des poignées(64, 76) est alors effectuée dans le sens de l'effet désiré mais jusqu'à la valeur maximale en-dessous du déplacement longitudinal sur les rails(3) des branches(4) sur lesquelles il est appliqué. Les dynamomètres(54, 55, 72, 73) placés sur ces éléments de mesure(43, 65) vont nous donner les valeurs des forces qui doivent être exercées par les ressorts de l'orthèse vertébrale interne dynamique. Dans le même temps, nous assistons à une réduction des forces demandées aux tiges de l'orthèse: les valeurs de ces forces étant toujours données par les dynamomètres(29, 30, 40, 41) des branches correctrices(4) fixées sur le cadre(1) par leur base(5).
Les tiges et les ressorts de l'orthèse sont alors choisis en fonction de ces données et leur insertion peut être effectuée.

La rotation en torsion des ressorts de l'orthèse et leur blocage par rapport à leurs points d'application sur les éléments d'ancrage, vont permettre d'exercer un certain effet de dérotation sur les vertèbres et donc réduire d'autant les forces demandées aux tiges de liaison dont le diamètre pourra être diminué, l'élasticité étant ainsi augmentée.

Dans le cas de pose d'un matériel d'ostéosynthèse, il n'est pas nécessaire d'obtenir une mesure des forces de correction: il faut donner aux moyens de liaison la forme nécessaire pour conserver la position de correction obtenue puis fixer ces derniers aux moyens d'ancrage.

L'intervention se termine par l'ablation du cadre(1) dont les extrémités correctrices sont désolidarisées des moyens d'ancrage, puis le cadre est libéré de ses bras articulés. La fermeture de l'incision est effectuée de la façon habituelle.

**Revendications**

1. Matériel ancillaire permettant d'exercer et de maintenir des contraintes sur une portion de la colonne vertébrale en vue de corriger et/ou de maintenir la forme et/ou les efforts exercés sur les vertèbres avant et pendant la pose d'une instrumentation rachidienne implantée, comprenant au moins deux extrémités d'action (6) destinées à coopérer respectivement avec au moins deux vertèbres distinctes, en s'appuyant, par leur branche correctrice correspondante (4), et par leur base (5) amovible, sur des rails longitudinaux et parallèles (3) d'un support (1) en forme de cadre, et également des moyens (13, 17, 43, 65) pour modifier et maintenir les positions relatives des extrémités d'action (6), caractérisé en ce que lesdits moyens (13, 17, 43, 65) sont adaptés pour modifier et maintenir les positions relatives des extrémités d'action (6) selon chacune, isolément ou en association, des trois directions orthogonales.

2. Matériel selon la revendication 1, caractérisé en ce qu'il comprend :

   - un support (1) en forme de cadre comprenant des barres longitudinales (3) adaptées pour s'étendre le long d'une colonne vertébrale,
   - des dispositifs de correction (4) montés coulissants sur lesdites barres longitudinales (3), chaque dispositif de correction (4) comprenant :

      . une base (5) de fixation dudit dispositif de correction (4) sur le support (1) tout en permettant des mouvements libres de déplacements longitudinaux,
      . une branche de correction (4) ayant une extrémité d'action (6) adaptée pour coopérer avec un côté correspondant d'une vertèbre, ladite branche correctrice (4) étant montée sur ladite base (5),
      . une tige de commande horizontale frontale (17) permettant des mouvements et des forces d'ajustement, par rapport à la base (5), de ladite extrémité d'action (6) selon une direction horizontale frontale orthogonale aux barres longitudinales (3), ladite tige de commande horizontale frontale (17) étant adaptée pour maintenir ladite extrémité d'action (6) dans la direction horizontale frontale après ajustement,

. une tige de commande horizontale sagittale (13) permettant des mouvements et des forces d'ajustement, par rapport à ladite base (5), de ladite extrémité d'action (6) selon une direction horizontale sagittale orthogonale aux barres longitudinales (3), cette tige de commande horizontale sagittale (13) étant adaptée pour maintenir l'extrémité d'action (6) dans la direction horizontale sagittale après ajustement.

3. Matériel selon l'une des revendications 1 et 2, caractérisé en ce qu'il comprend deux barres longitudinales (3) parallèles s'étendant de chaque côté de la colonne vertébrale, et en ce que ladite base (5) est fixée transversalement à chacune desdites barres (3) par des moyens de fixation adaptés pour autoriser des mouvements de libre coulissement longitudinaux de la base (5) le long des barres (3).

4. Matériel selon la revendication 3, caractérisé en ce que lesdits moyens de fixation soient adaptés pour autoriser le montage ou le démontage de chaque dispositif de correction (4) pendant l'opération chirurgicale.

5. Matériel selon l'une des revendications 3 et 4, caractérisé en ce que lesdits moyens de fixation comprennent, pour la fixation à chacune desdites barres (3) :

. un logement cylindrique (7),
. un cylindre (8) concentrique au logement cylindrique (7) et renfermé dans le logement cylindrique (7) de façon à être libre en rotation, et en ce que le logement cylindrique (7) et le cylindre (8) sont ouverts longitudinalement pour permettre la pénétration de chaque barre longitudinale dans le cylindre (8).

6. Matériel selon l'une des revendications 1 à 5, caractérisé en ce que le support (1) en forme de cadre comprend quatre barres longitudinales (3), adaptées pour pouvoir être disposées par deux de chaque côté d'une colonne vertébrale, de façon à permettre le montage de l'un des dispositifs de correction (4) et l'ancrage de l'une desdites extrémités d'action (6) sur chaque côté de chaque vertèbre.

7. Matériel selon l'une des revendications 1 à 6, caractérisé en ce que le support (1) en forme de cadre est un cadre rectangulaire comprenant deux petits côtés (2) supérieur et inférieur, et deux grands côtés longitudinaux (3), chaque côté longitudinal comprenant deux barres parallèles longitudinales (3), ce support (1) étant adapté pour s'étendre selon un plan frontal avec chacun des côtés longitudinaux (3) de chaque côté de la colonne vertébrale.

8. Matériel selon l'une des revendications 2 à 7, caractérisé en ce que chaque tige de commande horizontale frontale (17) comprend une poignée de manipulation (22), une butée axiale (40, 41) coopérant avec la branche correctrice (4), et au moins un filetage (31) coopérant avec un taraudage (32) solidaire de la base (5).

9. Matériel selon l'une des revendications 2 à 8, caractérisé en ce que la tige de contrôle frontale horizontale (17) comprend des moyens dynamométriques (40, 41), adaptés pour mesurer les forces exercées sur l'extrémité d'action (6) selon la direction horizontale frontale.

10. Matériel selon l'une des revendications 2 à 9, caractérisé en ce que la tige de commande horizontale frontale (17) comprend des moyens (35) de mesure des déplacements de l'extrémité d'action (6) selon la direction horizontale frontale.

11. Matériel selon l'une des revendications 2 à 10, caractérisé en ce que la tige de commande horizontale sagittale (13) comprend une poignée de manoeuvre (21), une butée axiale (29, 30) coopérant avec ladite branche correctrice (4), et au moins un filetage (14) coopérant avec un taraudage (15) solidaire de la base (5).

12. Matériel selon l'une des revendications 2 à 11, caractérisé en ce que la tige de commande horizontale sagittale (13) comprend des moyens dynamométriques (29, 30) adaptés pour mesurer les forces exercées sur l'extrémité d'action (6) selon la direction horizontale sagittale.

13. Matériel selon l'une des revendications 2 à 12, caractérisé en ce que la tige de commande horizontale sagittale (13) comprend des moyens (25) pour mesurer les déplacements de l'extrémité d'action (6) selon la direction horizontale sagittale.

14. Matériel selon l'une des revendications 1 à 13, caractérisé en ce qu'il comprend en outre une pince (43) ou pied à coulisse (65) dynamométrique comprenant : (i) deux tétons (48, 49, 68, 69) adaptés pour pouvoir être engagés dans des orifices (20) des bases (5) de deux dispositifs de correction (4) distincts, et (ii) des moyens dynamométriques (54, 55, 72, 73) adaptés pour mesurer les forces exercées entre les tétons (48, 49, 68, 69) selon la direction verticale des barres longitudinales (3).

15. Matériel selon la revendication 14, caractérisé en ce que la pince (43) ou pied à coulisse (65) dynamométrique comprend une poignée de manoeuvre (64, 76) et des moyens pour ajuster et maintenir la distance entre les tétons (48, 49, 68, 69).

**Claims**

1. Ancillary device allowing to apply and maintain stresses on a portion of the spinal column in view of correcting and/or maintaining the shape and/or the stresses on the vertebras before and during the placing of an implanted rachial instrumentation, comprising at least two action ends (6) intended for cooperating respectively with at least two separate vertebras while resting with the corresponding correction branch (4) and the detachable base (5) thereof on longitudinal and parallel rails (3) of a frame-shaped support (1), as well as means (13, 17, 43, 65) for altering and maintaining the relative positions of the action ends (6), characterized in that said means (13, 17, 43, 65) are adapted for altering and maintaining the relative positions of the action ends (6) along each of the three orthogonal directions, separately or in conjunction.

2. Device according to claim 1, characterized in that said device comprises:

   - a frame-shaped support (1) comprising longitudinal bars (3), said bars being adapted for extending along a spinal column,
   - correction devices (4) which are slidably mounted on said longitudinal bars (3), wherein each correction device (4) comprises:

     * a base (5) for fixing said correction device (4) on the support (1) while allowing free lengthwise displacement movements,
     * a correction branch (4) having one action end (6) adapted for cooperation with one corresponding side of a vertebra, wherein said correction branch (4) is mounted on said base (5),
     * one horizontal frontal control rod (17) allowing adjustment movements and forces of said action end (6) relative to the base (5) along a horizontal frontal direction which is orthogonal to the longitudinal bars (3), wherein said horizontal frontal control rod (17) is adapted for maintaining said action end (6) in the horizontal frontal direction once the adjustment is completed,
     * one horizontal sagittal control rod (13) allowing adjustment movements and forces of said action end (6) relative to the base (5) along a horizontal sagittal direction which is orthogonal to the longitudinal bars (3), wherein this horizontal sagittal control rod (13) is adapted for maintaining the action end (6) in the horizontal sagittal direction once the adjustment is completed,

3. Device according to one of the claims 1 and 2, characterized in that said device comprises a pair of parallel longitudinal bars (3) extending on both sides of the spinal column, and in that said base (5) is fixed transversally to each of said pair of bars (3) by means of fixation means which are adapted for allowing free lengthwise sliding movements of the base (5) along the bars (3).

4. Device according to claim 3, characterized in that said fixation means are adapted for allowing the mounting or dismounting of each correction device (4) during a surgical operation.

5. Device according to one of claims 3 and 4, characterized in that in view of the fixation on each of said bars (3), said fixation means comprise:

   - a cylindrical seating (7),
   - a cylinder (8) which is concentric relative to and enclosed in the cylindrical seating (7) so as to be freely rotatable, and in that the cylindrical seating (7) and cylinder (8) are open longitudinally in order to allow the insertion of each longitudinal bar (3) into the cylinder (8).

6. Device according to one of the claims 1 to 5, characterized in that the frame-shaped support (1) comprises four longitudinal bars (3) which are adapted for being able to be arranged in pairs on both sides of a spinal column, in order to allow the mounting of one of the correction devices (4) and the fixation of one of the action ends (6) on each side of each vertebra.

7. Device according to one of the claims 1 to 6, characterized in that the frame-shaped support (1) is a rectangular frame comprising two small, upper and lower sides (2) and two large longitudinal sides (3), wherein each longitudinal side comprises two parallel longitudinal bars (3), said support (1) being adapted for extending along a frontal plane with each of the longitudinal sides (3) extending on one respective side of the spinal column.

8. Device according to one of the claims 2 to 7, characterized in that each horizontal frontal control rod (17) comprises a manipulating handle (22), an axial abutment (40, 41) cooperating with the correction branch (4), and at least one threaded screw (31) cooperating with a threaded bore (32) fixedly attached to the base (5).

9. Device according to one of the claims 2 to 8, characterized in that the horizontal frontal control rod (17) comprises dynamometric means (40, 41) which are adapted for measuring the forces as applied on the action end (6) along the horizontal frontal direction.

10. Device according to one of the claims 2 to 9, characterized in that the horizontal frontal control rod (17) comprises means (35) for measuring the movements of the action end (6) along the horizontal frontal direction.

11. Device according to one of claims 2 to 10, characterized in that the horizontal sagittal control rod (13) comprises a manipulating handle (21), an axial abutment (29, 30) cooperating with said correction branch (4), and at least one threaded screw (14) cooperating with a threaded bore (15) fixedly attached to the base (5).

12. Device according to one of the claims 2 to 11, characterized in that the horizontal sagittal control rod (13) comprises dynamometric means (29, 30) which are adapted for measuring the forces as applied on the action end (6) along the horizontal sagittal direction.

13. Device according to one of the claims 2 to 12, characterized in that the horizontal sagittal control rod (13) comprises means (25) for measuring the movements of the action end (6) along the horizontal sagittal direction.

14. Device according to one of the claims 1 to 13, characterized in that said device further comprises a clamp (43) or dynamometric sliding caliper (65) comprising : (i) a pair of nipples (48, 49, 68, 69) adapted for engagement in openings (20) in the bases (5) of a pair of separate correction devices (4), and (ii) dynamometric means (54, 55, 72, 73) adapted for measuring the forces acting between nipples (48, 49, 68, 69) along the vertical direction of the longitudinal bars (3).

15. Device according to claim 14, characterized in that the clamp (43) or dynamometric sliding caliper (65) comprises a manipulating handle (64, 76), and means for adjusting and maintaining the distance between nipples (48, 49, 68, 69).

**Patentansprüche**

1. Hilfseinrichtung, die die Ausübung und die Aufrechterhaltung von Belastungen auf einen Abschnitt der Wirbelsäule im Hinblick auf eine Korrektur und/oder zur Aufrechterhaltung der Form und/oder der ausgeübten Belastung auf die Wirbel vor und während der Anbringung einer implantierten Rückgratsinstrumentierung erlaubt, und die mindestens zwei Aktionsenden (6) aufweist, die zur jeweiligen Kooperation mit mindestens zwei unterschiedlichen Wirbeln durch Abstützung durch ihren entsprechenden Korrektionsschenkel (4) und durch ihre abnehmbare Basis (5) auf longitudinalen und parallelen Schienen (3) eines rahmenförmigen Trägers (1) bestimmt sind, sowie Mittel (13, 17, 43, 65) zur Modifizierung und Aufrechterhaltung der relativen Lagen der Aktionsenden (6), dadurch gekennzeichnet, dass die Mittel (13, 17, 43, 65) zur Modifizierung und Aufrechterhaltung der relativen Lagen der Aktionsenden (6) in jeder der drei orthogonalen Richtungen, isoliert oder in Verbindung, angepaßt sind.

2. Einrichtung gemäß Anspruch 1, dadurch gekennzeichnet, dass die Einrichtung die folgende Merkmale umfaßt:

- einen Träger (1) in Form eines Rahmens, der longitudinale Holme (3) umfaßt, wobei die longitudinale Holme so angepaßt sind, dass sie sich in der Lange einer Wirbelsäule erstrecken,
- verschiebbar auf den longitudinalen Holmen (3) angebrachte Korrektionsgeräte (4), wobei jedes Korrektionsgerät (4)

  * eine Basis (5) zur Fixierung des Korrektionsgeräts (4) auf dem Träger (1), die trotzdem freie Bewegungen longitudinaler Ortswechsel erlaubt,
  * einen Korrektionsschenkel (4) mit einem Aktionsende (6), das so angepaßt ist, dass es mit einer entsprechenden Seite eines Wirbels zusammenwirkt, wobei der Korrektionsschenkel (4) auf die Basis (5) montiert ist,
  * einen horizontale, frontale Bedienungsstift (17), der im Verhältnis zur Basis (5) dem Aktionsende (6) auf den longitudinalen Holmen (3) entsprechend einer horizontalen, frontalen, orthogonalen Richtung Justierungsbewegungen und -kräfte erlaubt, wobei der horizontale, frontale Bedienungsstift (17) so angepaßt ist, dass er das Aktionsende (6) nach der Justierung in der horizontalen, frontalen Richtung festhält,
  * einen horizontalen, sagittalen Bedienungsstift (13), der im Verhältnis zur Basis (5) dem Aktionsende (6) auf den longitudinalen Holmen (3) entsprechend einer horizontalen, sagittalen, orthogonalen Richtung Justierungsbewegungen und -kräfte erlaubt, wobei dieser horizontale, sagittale Bedienungsstift (13) so angepaßt ist, dass er das Aktionsende (6) nach der Justierung in der horizontalen, sagittalen Richtung festhält,
umfaßt.

3. Einrichtung gemäß einem der Ansprüche 1 und 2, dadurch gekennzeichnet, dass die Einrichtung zwei parallele, longitudinale Holme (3), die sich an jeder Seite der Wirbelsäule befinden, umfaßt, und dass die Basis (5) transversal an jedem Holm (3) durch Fixierungsmittel befestigt ist, wobei die Fixierungsmittel so angepaßt sind, dass sie Bewegungen eines ungehinderten, longitudinalen Verschiebens der Basis (5) in der Länge der Holme (3) zulassen.

4. Einrichtung gemäß Anspruch 3, dadurch gekennzeichnet, dass die Fixierungsmittel so angepaßt sind, dass sie eine Montage oder Demontage jedes Korrektionsgerätes (4) während einer chirurgischen Operation zulassen.

5. Einrichtung gemäß einem der Ansprüche 3 und 4, dadurch gekennzeichnet, dass die Fixierungsmittel zur Fixierung an jedem Holm (3)

   - ein zylindrisches Lager (7),
   - einen relativ zum zylindrischen Lager (7) konzentrischen Zylinder (8), der frei rotierbar in dem zylindrischen Lager (7) eingeschlossen ist, und dass das zylindrische Lager (7) und der Zylinder (8) longitudinal offen sind, um das Eindringen jedes longitudinalen Holms in den Zylinder (8) zu ermöglichen,
   umfassen.

6. Einrichtung gemäß einem der Ansprüche 1 bis 5, dadurch gekennzeichnet, dass der rahmenförmige Träger (1) vier longitudinale Holme (3) umfaßt, die so angepaßt sind, dass sie zu zweit an jeder Seite einer Wirbelsäule so angebracht werden können, um die Montage eines der Korrektionsgeräten (4) und die Verankerung eines der Aktionsenden (6) an jeder Seite jedes Wirbels zuzulassen.

7. Einrichtung gemäß einem der Ansprüche 1 bis 6, dadurch gekennzeichnet, dass der rahmenförmige Träger (1) ein rechteckiger Rahmen ist, der zwei kleine Seiten (2), eine obere und eine untere, und zwei große, longitudinale Seiten (3) umfaßt, wobei jede longitudinale Seite zwei parallele, longitudinale Holme (3) umfaßt, und wobei dieser Träger (1) so angepaßt ist, dass er sich in einer frontalen Ebene mit jeder der longitudinalen Seiten (3) an jeder Seite der Wirbelsäule erstreckt.

8. Einrichtung gemäß einem der Ansprüche 2 bis 7, dadurch gekennzeichnet, dass jeder horizontale, frontale Bedienungsstift (17) einen Bedienungsgriff (22) umfaßt, sowie einen axialen Anschlag (40, 41), die mit dem Korrektionsschenkel (4) zusammenwirkt und mindestens ein Gewinde (31), das mit einem fest auf der Basis (5) gehaltenen Innengewinde (32) zusammenwirkt.

9. Einrichtung gemäß einem der Ansprüche 2 bis 8, dadurch gekennzeichnet, dass der horizontale, frontale Bedienungsstift (17) dynamometrische Mittel (40, 41) umfaßt, die zur Messung der auf das Aktionsende (6) in der horizontalen, frontalen Richtung ausgeübten Kräfte angepaßt sind.

10. Einrichtung gemäß einem der Ansprüche 2 bis 9, dadurch gekennzeichnet, dass der horizontale, frontale Bedienungsstift (17) Mittel (35) zur Messung der Verschiebungen des Aktionsendes (6) in der horizontalen, frontalen Richtung umfaßt.

11. Einrichtung gemäß einem der Ansprüche 2 bis 10, dadurch gekennzeichnet, dass der horizontale, sagittale Bedienungsstift (13) einen Bedienungsgriff (21) umfaßt, sowie einen axialen Anschlag (29, 30), die mit dem Korrektionsschenkel (4) zusammenwirkt und mindestens ein Gewinde (14), das mit einem fest auf der Basis (5) gehaltenen Innengewinde (15) zusammenwirkt.

12. Einrichtung gemäß einem der Ansprüche 2 bis 11, dadurch gekennzeichnet, dass der horizontale, sagittale Bedienungsstift (13) dynamometrische Mittel (29, 30) umfaßt, die zur Messung der auf das Aktionsende (6) in der horizontalen, sagittalen Richtung ausgeübten Kräfte angepaßt sind.

13. Einrichtung gemäß einem der Ansprüche 2 bis 12, dadurch gekennzeichnet, dass der horizontale, sagittale Bedienungsstift (13) Mittel (25) zur Messung der Verschiebungen des Aktionsendes (6) in der horizontalen, sagittalen Richtung umfaßt.

14. Einrichtung gemäß einem der Ansprüche 2 bis 13, dadurch gekennzeichnet, dass die Einrichtung zusätzlich eine Klemme (43) oder dynamometrische Schieblehre (65) umfaßt, die (i) zwei Nippel (48, 49, 68, 69), die zum Eingriff in Öffnungen (20) der Basen (5) von zwei unterschiedlichen Korrekturgeräten (4) angepaßt sind, sowie (ii) dynamometrische Mittel (54, 55, 72, 73), die zur Messung der zwischen den Nippeln (48, 49, 68, 69) in der vertikalen Richtung der longitudinalen Holme (3) ausgeübten Kräfte, umfaßt.

15. Einrichtung gemäß dem Anspruch 14, dadurch gekennzeichnet, dass die Klemme (43) oder dynamometrische Schieblehre (65) einen Bedienungsgriff (64, 76) und Mittel zur Justierung und Aufrechterhaltung des Abstands zwischen den Nippeln (48, 49, 68, 69) umfaßt.

fig. 1

fig. 2

fig. 3

fig.4

fig. 5

fig.6

1

4a

4a'

4b'

4b

4c'

4c

4d'

4d

4e'

4e

4f'

4f

3

2

fig.7